# EUROPÄISCHE PATENTANMELDUNG

(11) **EP 4 488 243 A1**
(43) Veröffentlichungstag der Anmeldung: **08.01.2025**
(21) Anmeldenummer: 23183404.5
(22) Anmeldetag: 04.07.2023
(51) Int. Cl.: C03C 3/095, C03C 4/00, C03C 8/02

(54) **SÄURESTABILE GLASFÜLLSTOFFE UND DIESE ENTHALTENDE SELBSTHAFTENDE DENTALE KOMPOSITZEMENTE MIT GUTER TRANSPARENZ**

(71) Anmelder: Ivoclar Vivadent AG, 9494 Schaan (LI)
(72) Erfinder: Ritzberger, Christian, 9472 Grabs (CH); Moszner, Norbert, 9495 Triesen (LI); Dittmer, Marc, 6800 Feldkirch (AT); Catel, Yohann, 9475 Sevelen (CH); Gianasmidis, Alexandros, 9436 Balgach (CH)
(74) Vertreter: Uexküll & Stolberg

(57) **Zusammenfassung**

Röntgenopaker Glasfüllstoff mit folgender Zusammensetzung (Gew.-%): SiO₂: 54,0 - 64,0; Al₂O₃: 10,0 - 25,0; B₂O₃: 0,0 - 10,0; La₂O₃: 0,1 - 10,0; WOs: 5,0 - 14,0; ZrO₂: 0,1 - 7,0; MgO: 1,0 - 7,9; CaO: 0,1 - 6,0; ZnO: 0 - 6,0; SrO: 0 - 6,0, und radikalisch polymerisierbare Zusammensetzung, die neben dem röntgenopaken Glasfüllstoff mindestens ein radikalisch polymerisierbares Monomer ohne Säuregruppe, mindestens ein säuregruppenhaltiges radikalisch polymerisierbares Monomer, und mindestens einen Initiator für die radikalische Polymerisation enthält. Der Glasfüllstoff zeichnet sich durch eine hohe Säurestabilität aus.

## Beschreibung

Die Erfindung betrifft säurestabile, röntgenopake Glasfüllstoffe und radikalisch polymerisierbare, selbsthaftende Komposite mit guter Transparenz, die sich insbesondere als Dentalwerkstoffe eignen, z.B. als dentale Zemente, Füllungskomposite oder Verblendmaterialen sowie zur Herstellung von Inlays, Onlays oder Kronen.

Komposite werden im Dentalbereich hauptsächlich zur Herstellung von direkten und indirekten Füllungen, d.h. als direkte und indirekte Füllungskomposite, sowie als Zemente eingesetzt. Die polymerisierbare organische Matrix der Komposite besteht meistens aus einer Mischung von Monomeren, Initiator-Komponenten und Stabilisatoren. Als Monomere werden gewöhnlich Mischungen von Dimethacrylaten eingesetzt, die auch monofunktionelle und funktionalisierte Monomere enthalten können. Häufig eingesetzte Dimethacrylate sind 2,2-Bis[4-(2-hydroxy-3-ethacryloyloxypropyl)-phenyl]propan (Bis-GMA) und 1,6-Bis-[2-methacryloyloxyethoxycarbonylamino]-2,2,4-tri-methylhexan (UDMA), die eine hohe Viskosität haben und Polymerisate mit guten mechanischen Eigenschaften und geringem Polymerisationsschrumpf ergeben. Als reaktive Verdünner finden vor allem Triethylenglycoldimethacrylat (TEGDMA), 1,10-Decandioldimethacrylat (D₃MA) oder Bis(3-methacryloyloxymethyl)tricyclo-[5.2.1.0_{2,6}]decan (DCP) Anwendung. Monofunktionelle Methacrylate, wie z.B. p-Cumylphenoxyethylenglycolmethacrylat (CMP-1E), eignen sich ebenfalls zur Reduzierung der Viskosität und bewirken darüber hinaus eine Verringerung der Netzwerkdichte und einen erhöhten Doppelbindungsumsatz.

Zur Herstellung selbsthaftender Komposite werden stark saure Haftmonomere eingesetzt, wie z.B. 10-Methacryloyloxydecyldihydrogenphosphat (MDP), das die Zahnhartsubstanz ätzt und durch Ionenbeziehung eine Haftung auf Schmelz/Dentin bewirkt. Haftmonomere verleihen Kompositen selbsthaftende Eigenschaften und ermöglichen damit den Einsatz der Komposite ohne eine Vorbehandlung der Zahnhartsubstanz mit einem Schmelz-Dentin-Adhäsiv, was deren Verwendung besonders attraktiv macht. Neben der organischen Matrix enthalten Komposite einen oder mehrere Füllstoffe, die meist mit einem polymerisationsfähigen Haftvermittler, wie z.B. 3-Methacryloyloxypropyltrimethoxysilan, oberflächenmodifiziert sind. Füllstoffe verbessern die mechanischen Eigenschaften (Festigkeit, Elastizitätsmodul, Abrasionsfestigkeit) und die Verarbeitungseigenschaften (Pastenkonsistenz, Stopfbarkeit) der Werkstoffe und verleihen ihnen Röntgenopazität.

Problematisch ist, dass saure Haftmonomere mit Füllstoffen häufig nachteilige Wechselwirkungen eingehen. Beispielsweise werden saure Haftmonomere durch die Bildung unlöslicher Salze an die Füllstoffoberfläche gebunden, oder sie bilden bei der Lagerung mit aus den Füllstoffen herausgelösten Ionen schwerlösliche Salze. Dies führt zu einer deutlichen Verringerung der Haftmonomerkonzentration in der Harzmatrix, was mit einer Verminderung oder sogar einem Verlust der Hafteigenschaften verbunden ist. Komposite mit sauren Haftmonomeren haben daher nur eine begrenzte Lagerstabilität.

Die Aushärtung methacrylatbasierender Dentalmaterialien erfolgt durch radikalische Polymerisation, wobei je nach Anwendungsgebiet Photoinitiatoren, thermische Initiatoren oder Redox-Initiatorsysteme eingesetzt werden. Dualhärtende Systeme enthalten eine Kombination von Photoinitiatoren und Redoxinitiatoren.

Kompositzemente enthalten in der Regel Redoxsysteme, weil diese auch dann eine ausreichende Härtung gewährleisten, wenn eine Lichthärtung wegen einer unzureichenden Durchstrahlung nicht möglich ist. Meist werden Redoxinitiatorsysteme eingesetzt, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) basieren. Da die Radikalbildung bei DBPO/Amin-basierenden Redoxinitiatorsystemen durch starke Säuren und damit auch durch stark saure Haftmonomere sehr beeinträchtigt wird, werden bevorzugt cumolhydroperoxidhaltige Redoxinitiatorsysteme in Kombination mit Thioharnstoffen, wie z.B. Acetylthioharnstoff, verwendet.

Um eine ausreichende Lagerstabilität der Redoxinitiatoren zu gewährleisten, werden Redox-Initiatorsystem-basierende Materialien meist als sog. 2-Komponenten-Systeme (2K) eingesetzt, wobei das Oxydationsmittel (Peroxid- oder Hydroperoxid) und das Reduktionsmittel (Amine, Sulfinsäuren, Barbiturate, Thioharnstoffe etc.) in räumlich getrennte Komponenten eingearbeitet werden. Diese werden erst kurz vor der Anwendung miteinander gemischt. Zum Mischen werden zunehmend Doppelschubspritzen verwendet, die getrennte zylindrische Kammern zur Aufnahme der Komponenten aufweisen. Die Komponenten werden mit zwei miteinander verbundenen Kolben gleichzeitig aus den Kammern herausgedrückt und in einer Düse miteinander gemischt. Um möglichst homogene Mischungen zu erhalten, ist es vorteilhaft, die Komponenten in etwa gleich großen Volumenanteilen miteinander zu mischen.

Herkömmliche Befestigungszemente, wie z.B. ZnO-Eugenol-Zemente, Zinkphosphat-Zemente, Glasionomer-Zemente (GIZ) und harzmodifizierte Glasionomer-Zemente (Resin Modified Glass Ionomer Cements: RMGI) eigenen sich nicht zur Anwendung mit Doppelschubspritzen, weil sie eine Pulverkomponente enthalten, was ein Mischen der Komponenten erheblich erschwert. Außerdem haben Glasionomerzemente nur eine geringe Transparenz und relativ schlechte mechanische Eigenschaften.

Konventionelle Glasionomer-Zemente (GIZ) enthalten als flüssige Komponente eine wässrige Lösung einer hochmolekularen Polyacrylsaure (PAA, zahlenmittlere Molmasse größer 30.000 g/mol) oder eines Copolymers vergleichbarer Molmasse aus Acrylsaure und Itaconsäure und als Pulverkomponente ein Calcium-Fluor-Aluminium-Glas. Sie härten nach dem Mischen der Komponenten durch eine rein ionische Ionomerbildung aus. Nachteilig an Glasionomerzementen sind ihre geringe Transparenz und ihre schlechten mechanischen Eigenschaften. RMGI enthalten zusätzlich hydrophile Monomere, wie z.B. 2-Hydroxyethylmethacrylat (HEMA). Ihre Aushärtung erfolgt sowohl durch eine Säure-Base-Reaktion als auch durch radikalische Polymerisation. Sie zeichnen sich gegenüber herkömmlichen GIZ durch eine verbesserte Biegefestigkeit aus.

Die US 8,053,490 B2 offenbart Fluorid freisetzende Dentalwerkstoffe, die einen Fluoroaluminiumsilikatglasfüllstoff (FAS) enthalten, der mit einer wässrigen Lösung eines säuregruppenhaltigen Monomers oder Oligomers umgesetzt wurde. Das säuregruppenhaltige Monomer oder Oligomer wird dabei an die Füllstoffoberfläche gebunden und soll eine Reaktion des Füllstoffs mit reaktiven Bestandteilen des Zements verhindern. Die Werkstoffe zeichnen sich durch eine geringe Transparenz und unbefriedigende mechanische Eigenschaften aus.

Die JP 20116030741 A1 offenbart Dentalwerkstoffe, die als Füllstoff ein Fluoroboroaluminiumsilikatglas enthalten, das mit einem Silan und einer polymeren Carbonsäure oberflächenmodifiziert ist. Die Füllstoffe weisen eine sphärische Struktur auf und sollen eine dauerhafte Freisetzung von Fluoridionen und anderen Ionen aufweisen. Die Dentalwerkstoffe zeigen keine Selbsthaftung auf Dentin und Zahnschmelz und haben nur unbefriedigende mechanische Eigenschaften.

Die US 2016/0324729 A1 offenbart Füllstoffe für Glasionomerzemente, deren Oberfläche zunächst silanisiert und dann mit einer ungesättigten Carbonsäure modifiziert wird, die über ein Siliciumatom an die Füllstoffoberflache gebunden wird. Die Carboxylgruppe der Carbonsäure soll mit den Bestandteilen des Glasionomerzements wechselwirken und den Zement auf diese Weise stabilisieren. Die Werkstoffe haben nur eine mäßige Transparenz und schlechte mechanische Eigenschaften.

Die CN 102976618 A offenbart kostengünstige Glasfüllstoffe für wasserbasierende Glasionomerzemente, die 25-35 Gew.-% Al₂O₃, 30-45 Gew.-% SiO₂, 2-8 Gew.-% Na₂O, 5-15 Gew.-% CaF₂ und 5-8 Gew.-% SrO und/oder CaO enthalten. Die Glaspulver werden bei Raumtemperatur mit Salzsäure oder Essigsaure behandelt und anschließend getrocknet. Sie sollen eine stabile Qualität und gute mechanische Eigenschaften haben und kontinuierlich Fluoridionen freisetzen. Die Glasionomerzemente haben nur eine geringe Transparenz und schlechte mechanische Eigenschaften.

Dai et al., Int. J. Nanomedicine 14 (2019) 9185, haben gezeigt, dass die Oberflächenbehandlung von basischen ZrO₂-Füllstoffen mit 10-Methacryloyloxydecyldihydrogenphosphat (MDP) die Biegefestigkeit von dentalen Kompositen verbessern kann und dass eine vorherige Beschichtung der ZrO₂-Partikel mit Zr(OH)₄ die Bindung von MDP an das Zirkonoxid verbessert. Dentalwerkstoffe auf der Basis der oberflächenbehandelten Füllstoffe sind nicht selbsthaftend und haben nur eine geringe Transparenz.

Gemäß US 8,071,662 B2 soll die Oberflächenmodifizierung von basischen Füllstoffen, wie z.B. ZrO₂, mit einer starken Säure, wie z.B. 3-Methacryloyloxypropylsulfonsäure, die Lagerstabilität von selbstätzenden Dentalwerkstoffen verbessern. Die offenbarten Dentalwerkstoffe sind nicht selbsthaftend und haben eine unbefriedigende Transparenz.

DE 24 46 546 A1 offenbart die Behandlung von natürlich vorkommendem Siliciumdioxid mit einer wasserhaltigen Mineralsäure zum Entfernen säurelöslicher Verunreinigungen. Das Siliciumdioxid soll sich als Füllstoff für dentale Zwecke eignen. Röntgenopake oder selbsthaftende Dentalwerkstoffe werden nicht offenbart.

Die US 2001/0034309 A1 offenbart die Behandlung anorganischer Füllstoffe mit Persäuren oder Persäuresalzen zum Entfernen organischer Substanzen, z.B. von Kohlenstoff. Durch das Entfernen organischer Stoffe soll die Bindung von Silanhaftvermittlern an die Füllstoffoberflache und damit die Einbindung der Füllstoffe in die organische Matrix von radikalisch polymerisierbaren Dentalwerkstoffen verbessert werden. Selbsthaftende Materialien werden nicht offenbart.

Der Erfindung liegt die Aufgabe zugrunde, säurestabile, röntgenopake Glasfüllstoffe und lagerstabile, selbsthaftende dentale Komposite mit guter Transparenz und guten mechanischen Eigenschaften bereitzustellen, die sich als 2-Komponentensysteme gut mit Doppelschubspritzen mischen und applizieren lassen. Die Komposite sollen sich insbesondere als dentale Befestigungszemente eignen, röntgenopak und einfach herstellbar sein.

Diese Aufgabe wird durch Glasfüllstoff mir der folgenden Zusammensetzung (Gew.-%): SiO₂: 54,0 - 64,0; Al₂O₃: 10,0 - 25,0; B₂O₃: 0,0 - 10,0; La₂O₃: 0,1 - 10,0; WO₃: 5,0 - 14,0; ZrO₂: 0,1 - 7,0; MgO: 1,0 - 7,9; CaO: 0,1 - 6,0; ZnO: 0 - 6,0; SrO: 0 - 6,0, und durch radikalisch polymerisierbare Zusammensetzungen gelöst, die mindestens ein radikalisch polymerisierbares Monomer ohne Säuregruppen, mindestens ein säuregruppenhaltiges, radikalisch polymerisierbares Monomer, mindestens einen Initiator für die radikalische Polymerisation und mindestens einen röntgenopaken Glasfüllstoff mit der angegebenen Zusammensetzung enthalten. Füllstoffhaltige Zusammensetzungen werden als Komposite bezeichnet.

Alle Prozentangaben beziehen sich wenn nicht anders angegeben auf die Gesamtmasse des Glases, wobei die Komponenten als Oxide berechnet werden, so wie es bei Gläsern und Glaskeramiken üblich ist.

Die erfindungsgemäßen Glasfüllstoffe enthalten keine Alkalimetalloxide bzw. -ionen, d.h. insbesondere kein LizO, Na₂O, K₂O, Rb₂O und Cs₂O, und vorzugsweise auch kein BaO.

Es wurde überraschend gefunden, dass röntgenopake Glasfüllstoffe mit der angegebenen Zusammensetzung besonders säurestabil sind, insbesondere gegenüber den zur Herstellung selbsthaftender Dentalwerkstoffe eingesetzten sauren Haftmonomeren, ohne dass sie einer Säurebehandlung oder einer anderen aufwendigen Oberflächenbehandlung unterzogen werden müssen. Sie ermöglichen eine vereinfachte Herstellung lagerstabiler Kompositwerkstoffe mit selbsthaftenden Eigenschaften.

Die röntgenopaken Glasfüllstoffe werden vorzugsweise ohne vorherige Säurebehandlung eingesetzt, d.h. insbesondere nicht mit einer organischen oder anorganischen Säure oder einer Lösung davon gewaschen. Gemäß einer besonders bevorzugten Ausführungsform der Erfindung werden die röntgenopaken Glasfüllstoffe auch ohne jede andere vorherige Oberflächenbehandlung eingesetzt, insbesondere ohne eine Oberflächenbehandlung gemäß dem in der Einleitung diskutierten Stand der Technik.

Die röntgenopaken Glasfüllstoffe können aber zur Verbesserung des Verbundes zwischen Füllstoff und Matrix silanisiert werden. Die Silanisierung erfolgt vorzugsweise mit einem radikalisch polymerisierbaren Silan, besonders bevorzugt mit einem methacrylatfunktionalisierten Silan und ganz besonders bevorzugt mit 3-Methacryloyloxypropyltrimethoxysilan (MEMO). Eine Silanisierung stellt erfindungsgemäß keine Oberflächenbehandlung im obigen Sinne dar.

Bevorzugte sind röntgenopake Glasfüllstoffe mit der folgende Zusammensetzung (Gew.-%): SiO₂: 55,0 - 63,0, besonders bevorzugt 55,0 - 62,0; Al₂O₃: 12,0 - 24,0, besonders bevorzugt 14,0 - 22,0; B₂O₃: 0,1 - 8,0, besonders bevorzugt 0,1 - 5,9; La₂O₃: 0,1 - 8,0, besonders bevorzugt 0,1 - 6,0; WO₃: 5,0 -13,0, besonders bevorzugt 5,0 - 12; ZrO₂: 0,1 - 6,0, besonders bevorzugt 0,1 - 5,0; MgO: 2,0 - 7,5, besonders bevorzugt 3,0 - 7,0; CaO: 0,1 - 4,0, besonders bevorzugt 0,1 - 3,0; ZnO: 0 - 6,0, besonders bevorzugt 0 - 4,0; SrO: 0 - 6,0, besonders bevorzugt 0 - 5,0.

Ganz besonders bevorzugt sind röntgenopake Glasfüllstoffe mit der folgenden Zusammensetzung (Gew.-%): SiOz: 56,0 - 60,0; Al₂O₃: 14,0 - 20,0; B₂O₃: 0,1 - 5,9; La₂O₃: 1,0 - 2,0; WO₃: 8,0 - 12; ZrO₂: 1,0 - 3,0; MgO: 5,0 - 7,0; CaO: 1,0 - 3,0; ZnO: 0; SrO: 0. Am meisten bevorzugt sind Glasfüllstoffe, bei denen die Summe von La₂O₃ + WO₃ in einem Bereich von 5, 0 bis 11,6 Gew.-% und die Summe von Al₂O₃ + La₂O₃ + WO₃ in einem Bereich von 23,6 - 31,6 Gew.-% liegen.

Die röntgenopaken Glasfüller haben vorzugsweise eine mittlere Partikelgösse von 0,2 bis 20 µm und besonders bevorzugt von 0,4 bis 5 µm. Wenn nicht anders angegeben, handelt es sich bei allen Partikelgrößen hierin um volumengemittelte Partikelgrößen (D₅₀-Werte), d.h. 50 % des Gesamtvolumens, das alle Partikel gemeinsam besitzen, ist in Partikeln enthalten, die einen Durchmesser kleiner als der angegebene Wert aufweisen.

Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 1 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25 °C, z.B. mit einem Malvern Zetasizer Nano ZS (Malvern Instruments, Malvern UK).

Im Fall aggregierter und agglomerierter Partikel kann die Primarteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 Å dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Die erfindungsgemäßen Zusammensetzungen enthalten vorzugsweise 10 Gew.-% bis 80 Gew.-%, besonders bevorzugt 20 bis 75 Gew.-% und ganz besonders bevorzugt 30 bis 70 Gew.-% des röntgenopaken Glasfüllers, bezogen auf die Gesamtmasse der Zusammensetzung.

Zusätzlich zu den röntgenopaken Glasfüllstoffen können die erfindungsgemäßen Zusammensetzungen weitere Füllstoffe enthalten.

Bevorzugte weitere Füllstoffe sind Metalloxide, besonders bevorzugt Mischoxide, die 60 bis 80 Gew.-% SiO₂ und mindestens eines der Metalloxide Yb₂O₃, ZnO, Ta₂O₅, Nb₂O₅ und/oder La₂O₃, vorzugsweise Yb₂O₃ und/oder ZnO enthalten, so dass sich die Gesamtmenge auf 100 % ergänzt. Mischoxide sind z.B. durch hydrolytische Cokondensation von Metallalkoxiden zugänglich. Die Metalloxide haben vorzugsweise eine mittlere Partikelgröße von 0,05 bis 10 µm, besonders bevorzugt von 0,1 bis 5 µm.

Weitere bevorzugte zusätzliche Füllstoffe sind pyrogene Kieselsäure oder Fällungskieselsäure mit einer Primärpartikelgröße von 0,01-0,15 µm sowie Quarz- oder Glaskeramikpulver mit einer Partikelgröße von 0,1 bis 15 µm, vorzugweise von 0,2 bis 5 µm, sowie Ytterbiumtrifluorid. Das Ytterbiumtrifluorid hat vorzugsweise eine 10 Partikelgröße von 80 bis 900 nm und besonders bevorzugt 100 bis 300 nm.

Außerdem sind als weitere Füllstoffe sog. Kompositfüller bevorzugt. Diese werden auch als Isofüller bezeichnet. Hierbei handelt es sich um splitterförmige Polymerisate, die ihrerseits einen Füllstoff enthalten, vorzugsweise pyrogenes SiO₂, Glasfüller und/oder Ytterbiumtrifluorid. Bevorzugt sind Polymerisate auf der Basis von Dimethacrylaten. Zur Herstellung der Isofüller werden der oder die Füllstoffe z.B. in eine Dimethacrylatharzmatrix eingearbeitet, die so erhaltene Kompositpaste anschließend thermisch polymerisiert und dann gemahlen.

Ein erfindungsgemäß bevorzugter Kompositfüller lässt sich beispielsweise durch thermische Härtung einer Mischung von Bis-GMA (8,80 Gew.-%), UDMA (6,60 Gew.-%), 1,10-Decandioldimethacrylat (5,93 Gew.-%), Dibenzoylperoxid + 2,6-Di-tert.-butyl-4-methylphenol (zusammen 0,67 Gew.-%), Glasfüller (mittlere Korngröße 0,4 µm; 53,0 Gew.-%) und YbF₃ (25,0 Gew.-%) und anschließendes Vermahlen des gehärteten Materials auf die gewünschte Korngröße herstellen. Alle Prozentangaben beziehen sich auf die Gesamtmasse des Kompositfüllers.

Als weitere Füllstoffe können auch sog. inertisierte Füllstoffe eingesetzt werden. Dabei handelt es sich um Glasfüllstoffe, deren Oberfläche mit einer Diffusionsbarriereschicht, z.B. auf Sol-Gel-Basis, oder mit einer Polymerschicht, z.B. aus PVC, beschichtet ist. Bevorzugt sind die in der EP 2 103 296 A1 beschrieben Füllstoffe.

Zur Verbesserung des Verbundes zwischen Füllstoff und Matrix sind die weiteren Füllstoffe vorzugsweise mit methacrylatfunktionalisierten Silanen, wie z.B. 3-Methacryloyloxypropyltrimethoxysilan, oberflächenmodifiziert.

Die Gesamtmenge an weiteren Füllstoffen beträgt vorzugsweise 0,1 bis 25 Gew.-%, besonders bevorzugt 0,2 bis 20 Gew.-% und ganz besonders bevorzugt 0,3 bis 15 Gew.-%, jeweils bezogen auf die Gesamtmasse der Zusammensetzung. Erfindungsgemäß besonders bevorzugt sind Zusammensetzungen, die 0,1 bis 25 Gew.-%, bevorzugt 1 bis 20 Gew.-% und besonders bevorzugt 2 bis 15 Gew.-% eines oder mehrerer der genannten Metalloxide, pyrogene Kieselsäure, Fällungskieselsäure oder einer Mischung davon enthalten, bezogen auf die Gesamtmasse der Zusammensetzung.

Die erfindungsgemäßen Zusammensetzungen enthalten mindestens ein radikalisch polymerisierbares Monomer ohne Säuregruppen und mindestens ein radikalisch polymerisierbares Monomer mit Säuregruppen. Erfindungsgemäß sind Zusammensetzungen bevorzugt, die Monomere mit und Monomere ohne Säuregruppe in einem Gewichtsverhältnis von 1:5 bis 1:36, besonders bevorzugt von 1:6 bis 1:25 und ganz besonders bevorzugt von 1:7 bis 1:20 enthalten.

Erfindungsgemäß bevorzugt sind Zusammensetzungen, die mindestens ein polyfunktionelles Monomer und besonders bevorzugt mindestens ein monofunktionelles und mindestens ein polyfunktionelles Monomer enthalten. Unter polyfunktionellen Monomeren werden Monomere mit zwei oder mehr, vorzugsweise 2 bis 4 und insbesondere 2 radikalisch polymerisierbaren Gruppen verstanden. Monofunktionelle Monomere weisen dementsprechend nur eine radikalisch polymerisierbare Gruppe auf. Polyfunktionelle Monomere haben vernetzende Eigenschaften und werden daher auch als Vernetzermonomere bezeichnet. Bevorzugte radikalisch polymerisierbare Gruppen sind (Meth)acrylat-, (Meth)acrylamid- und Vinylgruppen.

Erfindungsgemäß wird zwischen säuregruppenhaltigen Monomeren und Monomeren unterschieden, die keine Säuregruppen enthalten. Bevorzugte Monomere dieser Typen werden im folgenden definiert.

### Monomere ohne Säuregruppe

Bevorzugte Monomere ohne Säuregruppen sind (Meth)acrylate, insbesondere monofunktionelle und polyfunktionelle Methacrylate und ganz besonders bevorzugt eine Mischung davon. Als polyfunktionelle (Meth)acrylate sind difunktionelle Methacrylate bevorzugt. Bevorzugte monofunktionelle (Meth)acrylate sind Benzyl-, Tetrahydrofurfuryl- oder Isobornyl(meth)acrylat, p-Cumyl-phenoxyethylenglycolmethacrylat (CMP-1E) und 2-([1,1'-Biphenyl]-2-oxy)ethylmethacrylat (MA-836), Tricyclodecanmethyl(meth)-acrylat, 2-(2-Biphenyloxy)ethyl(meth)acrylat. Besonders geeignet sind CMP-1E und MA-836.

Gemäß einer Ausführungsform enthalten die erfindungsgemäßen Zusammensetzungen vorzugsweise mindestens ein funktionalisiertes monofunktionelles (Meth)acrylat. Unter funktionalisierten Monomeren werden solche Monomere verstanden, die neben mindestens einer radikalisch polymerisierbaren Gruppe mindestens eine funktionelle Gruppe tragen, vorzugsweise eine Hydroxylgruppe. Bevorzugte funktionalisierte Mono(meth)acrylate sind 2-Hydroxyethyl- und Hydroxyethylpropyl(meth)acrylat sowie 2-Acetoxyethylmethacrylat. Besonders bevorzugt ist 2-Hydroxyethylmethacrylat. Die unten genannten säuregruppenhaltigen Monomere sind keine funktionalisierten Monomere im Sinne der Erfindung.

Bevorzugte di- und polyfunktionelle (Meth)acrylate sind Bisphenol-A-dimethacrylat, Bis-GMA (ein Additionsprodukt aus Methacrylsäure und Bisphenol-A-diglycidylether), ethoxy- oder propoxylierte Bisphenol-A-dimethacrylate, wie z.B. das Bisphenol-A-dimethacrylat SR-348c (Sartomer) mit 3 Ethoxygruppen oder 2,2-Bis[4-(2-methacryloy-loxypropoxy)phenyl]propan, Urethane aus 2-Hydroxymethyl)acrylsauremethylester und Diisocyanten, wie 2,2,4-Trimethylhexamethylendiisocyanat oder Isophorondiisocyanat, UDMA (ein Additionsprodukt aus 2-Hydroxyethylmethacrylat und 2,2,4-Trimethylhexamethylen-1,6-diisocyanat), Tetramethylxylylendiurethanethylenglykoldi-(meth)acrylat bzw. Tetramethylxylylendiurethan-2-methylethylenglykoldi-(meth)acrylat (V-380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tricyclo-[5.2.1.0_{2,6}]decan (DCP), Polyethylenglycol- oder Polypropylenglycoldimethacrylate, wie z.B. Polyethylenglycol-200-dimethacrylat oder Polyethylenglycol-400-dimethacrylat (PEG-200- oder PEG-400-DMA) oder 1,12-Dodecandioldimethacrylat. Besonders bevorzugt sind Bis-GMA, UDMA, V-380, Triethylenglycoldimethacrylat (TEGDMA) und PEG-400-DMA (NK-Ester 9G).

Das Monomer Tetramethylxylylendiurethanethylenglykoldi(meth)acrylat bzw. Tetra-methylxylylendiurethan-2-methylethylenglykoldiurethandi(meth)acrylat (V-380) weist die folgende Formel auf:

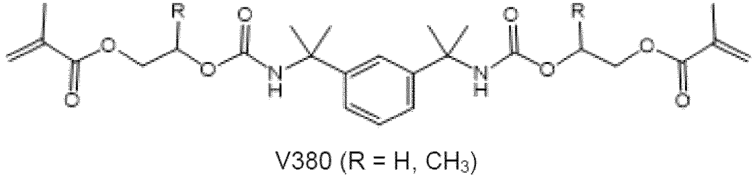

In der gezeigten Formel sind die Reste R unabhängig voneinander jeweils H oder CH₃, wobei die Reste die gleiche Bedeutung oder unterschiedliche Bedeutungen haben können. Vorzugsweise wird eine Mischung eingesetzt, die Moleküle, in denen beide Reste H sind, Moleküle, in denen beide Reste CH₃ sind, und Moleküle enthält, in denen ein Rest H und der andere Rest CH₃ ist, wobei das Verhältnis von H zu CH₃ vorzugsweise 7:3 ist. Eine solche Mischung ist beispielsweise durch Reaktion von 1,3-Bis(1-isocyanato-1-methylethyl)benzol mit 2-Hydroxypropylmethacrylat und 2-Hydroxyethylmethacrylat erhältlich.

Weitere bevorzugte difunktionelle Monomere sind radikalisch polymerisierbare Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, sowie Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsaurechlorid synthetisiert werden können. Besonders bevorzugt ist N,N'-Diethyl-1,3-bis(acrylamido)propan (V-392). Diese Monomere zeichnen sich durch eine hohe Hydrolysestabilität aus.

### Säuregruppenhaltige Monomere und Oligomere

Die erfindungsgemäßen Zusammensetzungen enthalten mindestens ein saures, radikalisch polymerisierbares Monomer. Unter sauren Monomeren werden Monomere verstanden, die mindestens eine Säuregruppe enthalten, vorzugsweise eine Phosphorsäureester-, Phosphonsäure- oder Carboxygruppe. Saure Monomere werden hierin auch als Haftmonomere bezeichnet. Erfindungsgemäß sich solche Zusammensetzungen besonders bevorzugt, die mindestens ein stark saures Monomer enthalten. Unter stark sauren Monomeren werden Monomere mit einem pKₐ-Wert von 0,5 bis 4,0, besonders bevorzugt 1,0 bis 3,5 und ganz besonders bevorzugt 1,5 bis 2,5 bei Raumtemperatur verstanden.

Geeignete säuregruppenhaltige Monomere sind COOH-gruppenhaltige polymerisationsfähige Monomere, vorzugsweise mit einem pKₐ-Wert im Bereich von 2,0 bis 4,0. Bevorzugt sind 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure. Methacrylsäure (pKₐ = 4,66) ist aufgrund ihrer geringen Haftung auf der Zahnhartsubstanz ausgeschlossen.

Bevorzugte säuregruppenhaltige Monomere sind Phosphorsäureester- und Phosphonsäuremonomere, vorzugsweise mit einem pKₐ-Wert im Bereich von 0,5 bis 3,5. Besonders bevorzugte sind 2-Methacryloyloxyethylphenylhydrogenphosphat, 10-Methacryloyloxydecyl-dihydrogenphosphat (MDP), Glycerindimethacrylatdihydrogenphosphat oder Dipentaerythritpentamethacryloyloxyphosphat, 4-Vinylbenzylphosphonsäure, 2-[4-(Dihydroxyphosphoryl)-2-oxa-butyl]-acrylsäure oder hydrolysestabile Ester, wie z.B. 2-[4-(Dihydroxyphosphoryl)-2-oxa-butyl]-acrylsäure-2,4,6-trimethylphenylester. Ganz besonders bevorzugt sind MDP, 2-Methacryloyloxyethylphenyl-hydrogenphosphat und Glycerindimethacrylatdihydrogenphosphat.

Die erfindungsgemäßen Zusammensetzungen können neben dem säuregruppenhaltigen Monomer zusätzlich ein oder mehrere saure radikalisch polymerisierbare Oligomere enthalten. Zusammensetzungen, die keine sauren radikalisch polymerisierbaren Oligomere enthalten, sind jedoch bevorzugt. Unter Oligomeren werden Polymere mit einem Polymerisationsgrad Pₙ von 2 bis 100 verstanden (Pₙ = Mₙ / Mᵤ; Mₙ: zahlenmittlere Polymermolmasse, Mu: Molmasse der Monomereinheit). Saure, radikalisch polymerisierbare Oligomere weisen mindestens eine Säuregruppe, vorzugsweise Carboxygruppe, und mindestens eine radikalisch polymerisierbare Gruppe, vorzugsweise mindestens eine (Meth)acrylat- und besonders bevorzugt mindestens eine Methacrylatgruppe auf.

Erfindungsgemäß bevorzugte säuregruppenhaltige Oligomere sind oligomere Carbonsäuren, wie z.B. Polyacrylsäure, vorzugsweise mit einer zahlenmittleren Molmasse Mₙ kleiner 7200 g/mol, bevorzugt kleiner 7000 g/mol und besonders bevorzugt kleiner 6800 g/mol, wobei Mₙ bevorzugt in einem Bereich von 800 bis 7200 g/mol, besonders bevorzugt von 500 bis 7000 g/mol und ganz besonders bevorzugt von 500 bis 6800 g/mol liegt. Besonders bevorzugt sind oligomere Carbonsäuren mit (Meth)acrylatgruppen. Diese sind z.B. durch Umsetzung von oligomerer Polyacrylsäure mit Glycidylmethacrylat oder 2-Isocyanatoethylmethacrylat erhältlich.

Wenn nicht anders angegeben, handelt es sich hierin bei der Molmasse von Oligomeren und Polymeren um die zahlenmittlere Molmasse Mn, die mittels Gelpermeationschromatographie (GPC) bestimmt wird.

Die Gelpermeationschromatographie (GPC) ist eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards. Als Kalibrierstandard werden vorzugsweise engverteile Polystyrolstandards eingesetzt. Diese sind kommerziell erhältlich. Als Trennmaterial werden Styrol-Divinylbenzol-Säulen und Tetrahydrofuran (THF) als Elutionsmittel verwendet. Styrol-Divinylbenzol-Säulen eignen sich für organische lösliche synthetische Polymere. Die Messung erfolgt mit verdünnten Lösungen (0,05 - 0,2 Gew.-%) der zu untersuchenden Polymere.

Alternativ kann die zahlenmittlere Molmasse mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmt werden. Hierbei handelt es sich um absolute Methoden, die keine Kalibrierstandards erfordern. Es werden Konzentrationsreihen von 4 bis 6 verdünnten Polymerlösungen mit Konzentrationen von 0,005 bis 0,10 mol/kg untersucht und dann die gemessenen Werte auf eine Konzentration von 0 mol/kg extrapoliert.

Die erfindungsgemäßen Zusammensetzungen enthalten vorzugsweise Wasser. Es wurde gefunden, dass ein Wassergehalt von 1 bis 7 Gew.-%, besonders bevorzugt 1 bis 5 Gew.-%, jeweils bezogen auf die Gesamtmasse der Zusammensetzung, eine Verbesserung der Haftwirkung an Dentin und Zahnschmelz bewirkt.

Die erfindungsgemäßen Zusammensetzungen enthalten außerdem mindestens einen Initiator zur Initiierung der radikalischen Polymerisation, vorzugsweise einen Photoinitiator. Bevorzugte Photoinitiatoren sind Benzophenon, Benzoin sowie deren Derivate, α-Diketone oder deren Derivate, wie 9,10-Phenanthrenchinon, 1-Phenylpropan-1,2-dion, Diacetyl und 4,4'-Dichlorbenzil. Besonders bevorzugt werden Campherchinon (CC) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin verwendet. Weiter bevorzugt sind Norrish-Typ-I-Photoinitiatoren, vor allem Acyl- oder Bisacylphosphinoxide und ganz besonders Monoacyltrialkylgermanium-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman oder Tetrakis(o-methyl-benzoyl)german. Dabei lassen sich auch Mischungen der verschiedenen Photoinitiatoren einsetzen, wie z.B. Bis(4-methoxybenzoyl)diethylgerman oder Tetrakis(o-methylbenzoyl)german in Kombination mit Campherchinon und 4-Dimethylaminobenzoesäureethylester.

Weiter bevorzugt sind Zusammensetzungen, die einen Redoxinitiator zur Initiierung der radikalischen Polymerisation enthalten, vorzugsweise einen Redoxinitiator auf der Basis eines Oxidationsmittels und eines Reduktionsmittels. Bevorzugte Oxidationsmittel sind Peroxide und insbesondere Hydroperoxide. Ein besonders bevorzugtes Peroxid ist Benzoylperoxid. Bevorzugte Hydroperoxide sind die in EP 3 692 976 A1 offenbarten geruchsarmen Cumolhydroperoxid-Derivate, die in EP 21315089.9 offenbarten oligomeren CHP-Derivate und insbesondere 4-(2-Hydroperoxypropan-2-yl)phenyl-propionat und Cumolhydroperoxid (CHP).

Bevorzugte Reduktionsmittel zur Kombination mit Peroxiden sind tertiäre Amine, wie N,N-Dimethyl-p-toluidin, N,N-Dihydroxyethyl-p-toluidin, p-Dimethylaminobenzoesäureethylester oder andere aromatische Dialkylamine, Ascorbinsäure, Sulfinsäuren, Thiole und/oder Hydrogensilane.

Bevorzugte Reduktionsmittel zur Kombination mit Hydroperoxiden sind Thioharnstoff-derivate, insbesondere die in der EP 1 754 465 A1 in Absatz [0009] aufgeführten Verbindungen. Besonders bevorzugt sind Methyl-, Ethyl-, Allyl-, Butyl-, Hexy, Octyl-, Benzyl-, 1,1,3-Trimethyl-, 1,1-Diallyl, 1,3-Diallyl-, 1-(2-Pyridyl-2-thioharnstoff, Acetyl-, Propanoyl-, Butanoyl-, Pentanoyl-, Hexanoyl-, Heptanoyl-, Octanoyl, Nonanoyl, Decanoyl, Benzoylthioharnstoff und Mischungen davon. Ganz besonders bevorzugt sind Acetyl-, Allyl-, Pyridyl- und Phenylthioharnstoff sowie Hexanoylthioharnstoff und Mischungen davon sowie polymerisationsfähige Thioharnstoff-Derivate, wie z.B. N-(2-Methacryloyloxyethoxysuccinoyl)-thioharnstoff und N-(4-Vinylbenzoyl)-thioharnstoff). Außerdem kann vorteilhaft eine Kombination von einem oder mehreren der genannten Thioharnstoffderivate mit einem oder mehreren Imidazolen eingesetzt werden. Bevorzugte Imidazole sind 2-Mercapto-1-methylimidazol oder 2-Mercaptobenzimidazol.

Die erfindungsgemäßen Zusammensetzungen können neben mindestens einem Hydroperoxid und mindestens einem Thioharnstoffderivat außerdem zusätzlich mindestens eine Übergangsmetallverbindung zur Beschleunigung der Härtung enthalten. Erfindungsgemäß geeignete Übergangsmetallverbindungen sind insbesondere Verbindungen, die sich von Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Zusammensetzungen, die mindestens eine Kupferverbindung enthalten, sind besonders geeignet. Die Übergansmetallverbindungen werden vorzugsweise in katalytische Mengen eingesetzt, besonders bevorzugt in einer Menge von 10 bis 200 ppm. Diese führen zu keinen Verfärbungen der Dentalmaterialien. Aufgrund der guten Monomerlöslichkeit werden die Übergangsmetalle vorzugsweise in Form ihrer Acetylacetonate, 2-Ethylhexanoate oder THF-Addukte eingesetzt. Bevorzugt sind weiterhin ihre Komplexe mit mehrzähnigen Liganden wie z.B. 2-(2-Aminoethylamino)ethanol, Triethylentetramin, Dimethylglyoxim, 8-Hydroxychinolin, 2,2'-Bipyridin oder 1,10-Phenanthrolin. Ein erfindungsgemäß besonders geeigneter Initiator ist eine Mischung von Cumolhydroperoxid (CHP) mit mindestens einem der oben genannten Thioharnstoffderivate und Kupfer(II)-acetylacetonat. Erfindungsgemäß sind Zusammensetzungen bevorzugt, die keine Vanadiumverbindungen enthalten.

Die erfindungsgemäßen Zusammensetzungen enthalten vorzugsweise keine Barbitursäure oder Barbitursäure-Derivate wie 1,3,5-Trimethylbarbitursäure, 1-Benzyl-5-phenylbarbitursäure, 5-Butylbarbitursäure oder 1-Cylohexyl-5-ethylbarbitursäure. Zusammensetzungen, die Barbiturate enthalten, weisen eine unbefriedigende Lagerstabilität auf, weil Barbiturate durch Oxidation mit Luftsauerstoff polymerisationsauslösende Radikale bilden. Außerdem haben Barbiturate nachteilige physiologische Wirkungen wie Bradykardie, Hypotonie oder Blutstörungen.

Die erfindungsgemäßen Zusammensetzungen können außerdem zusätzlich weitere Additive enthalten, vor allem Stabilisatoren, Farbmittel, Phasentransferkatalysatoren, mikrobizide Wirkstoffe, fluoridionenabgebende Additive, wie z.B. Fluorid-Salze, insbesondere NaF oder Ammoniumfluorid, oder Fluorsilane, optische Aufheller, Weichmacher und/oder UV-Absorber.

Erfindungsgemäß sind solche Zusammensetzungen besonders bevorzugt, die die folgenden Bestandteile enthalten:
a) 10 bis 80 Gew.-%, bevorzugt 20 bis 75 Gew.-% und besonders bevorzugt 30 bis 70 Gew.-% mindestens eines erfindungsgemäßen röntgenopaken Glasfüllstoffs,
b) ggf. 0,1 bis 25 Gew.-%, bevorzugt 1 bis 20 Gew.-% und besonders bevorzugt 2 bis 15 Gew.-% eines oder mehrerer weiterer Füllstoffe,
c) 1 bis 15 Gew.-%, bevorzugt 2 bis 12 Gew.-% und besonders bevorzugt 3 bis 10 Gew.-% mindestens eines säuregruppenhaltigen Monomers,
d) 5 bis 40 Gew.-%, bevorzugt 8 bis 30 Gew.-% oder 8 bis 29 Gew.-% und besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,1 bis 8 Gew.-%, bevorzugt 0,5 bis 6 Gew.-% und besonders bevorzugt 1 bis 5 Gew.-% eines Initiators für die radikalische Polymerisation,
h) 0 bis 20 Gew.-%, bevorzugt 0,2 bis 10 Gew.-% und besonders bevorzugt 1 bis 7 Gew.-% an Wasser und
i) 0,01 bis 5 Gew.-%, bevorzugt 0,1 bis 3 Gew.-% und besonders bevorzugt 0,1 bis 2 Gew.-% eines oder mehrerer Additive, jeweils bezogen auf die Gesamtmasse der Zusammensetzung.

Der Initiator kann ein Redoxinitiator, ein Photoinitiator oder ein Kombination davon sein. Die genannten Mengenangaben enthalten sämtliche Initiatorbestandteile, d.h. die Initiatoren selbst und ggf. Reduktionsmittel, Übergangsmetallverbindung etc. Erfindungsgemäß sind Zusammensetzungen bevorzugt, die mindestens einen Redoxinitiator oder mindestens einen Redoxinitiator und mindestens einen Photoinitiator enthalten.

Zusammensetzungen, die einen Redoxinitiator enthalten, werden auch als selbsthärtend bezeichnet. Sie werden vorzugsweise in der Form von zwei räumlich getrennten Komponenten, d.h. als 2-Komponenten-System (2K System), eingesetzt. Oxidations- und Reduktionsmittel werden dabei in separate Komponenten der Zusammensetzung eingebarbeitet. Eine Komponente, die sog. Katalysatorpaste, enthält das Oxidationsmittel, vorzugsweise ein Peroxid oder Hydroperoxid, und die zweite Komponente, die sog. Basis-Paste, das entsprechende Reduktionsmittel und ggf. einen Photoinitiator und ggf. katalytische Mengen einer Übergansmetallverbindung. Die Polymerisation wird durch das Mischen der Komponenten gestartet. Zusammensetzungen, die sowohl einen Redox- als auch einen Photoinitiator enthalten, werden als dualhärtend bezeichnet.

Erfindungsgemäß sind 2-Komponenten-Systeme bevorzugt. Sie sind vorzugsweise selbsthärtend oder dualhärtend. Die Pasten werden kurz vor der Anwendung, vorzugsweise mit einer Doppelschubspritze, miteinander gemischt. Die Katalysatorpaste weist vorzugsweise die folgende Zusammensetzung auf:
a) 10 bis 80 Gew.-%, bevorzugt 20 bis 75 Gew.-% und besonders bevorzugt 30 bis 70 Gew.-% mindestens eines säurestabilen röntgenopaken Glasfüllstoffs,
b) ggf. 0,1 bis 25 Gew.-%, bevorzugt 1 bis 20 Gew.-% und besonders bevorzugt 2 bis 15 Gew.-% eines oder mehrerer weiterer Füllstoffe,
c) 2 bis 30 Gew.-%, bevorzugt 4 bis 24 Gew.-% und besonders bevorzugt 6 bis 20 Gew.-% mindestens eines säuregruppenhaltigen Monomers,
d) 5 bis 40 Gew.-%, bevorzugt 8 bis 30 Gew.-% oder 8 bis 29 Gew.-% und besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,01 bis 16 Gew.-%, bevorzugt 0,2 bis 12 Gew.-% und besonders bevorzugt 0,5 bis 10 Gew.-% mindestens eines Peroxids und/oder Hydroperoxids ggf. mindestens eines Photoinitiators,
h) 0 bis 20 Gew.-%, bevorzugt 0,2 bis 10 Gew.-% und besonders bevorzugt 1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive, jeweils bezogen auf die Gesamtmasse der Katalysatorpaste.

Die Basis-Paste weist vorzugsweise folgende Zusammensetzung auf:
a) 10 bis 80 Gew.-%, bevorzugt 20 bis 75 Gew.-% und besonders bevorzugt 30 bis 70 Gew.-% mindestens eines röntgenopaken Glasfüllstoffs,
b) ggf. 0,1 bis 25 Gew.-%. bevorzugt 1 bis 20 Gew.-% und besonders bevorzugt 2 bis 15 Gew.-% eines oder mehrerer weiterer Füllstoffe,
d) 5 bis 40 Gew.-%, bevorzugt 8 bis 30 Gew.-% oder 8 bis 29 Gew.-% und besonders bevorzugt 10 bis 25 Gew.-% mindestens eines polyfunktionellen Monomers ohne Säuregruppen,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,01 bis 16 Gew.-%, bevorzugt 0,3 bis 12 Gew.-% und besonders bevorzugt 1 bis 10 Gew.-% mindestens eines geeigneten Reduktionsmittels und ggf. mindestens eines Photoinitiators,
h) 0 bis 20 Gew.-%, bevorzugt 0,2 bis 10 Gew.-% und besonders bevorzugt 1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive, jeweils bezogen auf die Gesamtmasse der Basis-Paste.

Zur Anwendung werden die Katalysator- und Basis-Paste vorzugsweise in etwa gleichen Anteilen miteinander gemischt. Sie eignen sich daher besonders zur Anwendung mit einer Doppelschubspritze. Doppelschubspritzen weisen zwei getrennte zylindrische Kammern zur Aufnahme von Basis- und Katalysatorpaste auf. Die Komponenten werden mit zwei miteinander verbundenen Kolben gleichzeitig aus den Kammern herausgedruckt und vorzugsweise durch eine Mischkanüle gepresst und darin miteinander gemischt. Zum Herausdrücken der Pasten kann die Spritze in einen sogenannten Handdispenser eingesetzt werden, der die Handhabung der Spritzen erleichtert.

Es wurde überraschend gefunden, dass die erfindungsgemäßen röntgenopaken Glasfüllstoffe auch ohne vorherige Oberflächenbehandlung eine hohe Säurestabilität aufweisen, insbesondere gegenüber den zur Herstellung selbsthaftender Dentalwerkstoffe eingesetzten sauren Haftmonomeren und Oligomeren. Sie können direkt mit den übrigen Bestandteilen der Werkstoffe gemischt werden und ergeben lagerstabile Kompositwerkstoffe mit selbsthaftenden Eigenschaften. Bei der Lagerung nimmt der Gehalt an säuregruppenhaltigen, radikalisch polymerisierbaren Monomeren nur sehr langsam ab, und die Zusammensetzungen zeigen auch nach längerer Aufbewahrung eine hohe Haftung an der Zahnhartsubstanz und insbesondere an Dentin.

Die erfindungsgemäßen Zusammensetzungen zeichnen sich außerdem durch eine verbesserte Transparenz aus. Diese ist vorzugsweise größer als 10 %. Sie eignen sich besonders als Dentalwerkstoffe zur intraoralen Anwendung durch den Zahnarzt zur Restauration geschädigter Zähne (therapeutische Anwendung), insbesondere als dentale Zemente, Beschichtungs- oder Verblendmaterialien, Fällungskomposite und ganz besonders als Befestigungszemente. Die Transparenz wird mittels eines Spektrophotometers, beispielsweise einem Konika-Minolta Spektrophotometer CM-5, an 1 mm dicken, auf Hochglanz polierten Prüfkörpern in Transmission gemessen.

Zur Behandlung geschädigter Zähne werden diese vorzugsweise in einem ersten Schritt durch den Zahnarzt präpariert. Anschließend wird mindestens eine erfindungsgemäße Zusammensetzung auf oder in den präparierten Zahn appliziert. Danach kann die Zusammensetzung direkt gehärtet werden, vorzugsweise durch Bestrahlen mit Licht einer geeigneten Wellenlänge, beispielsweise bei der Versorgung von Kavitäten. Alternativ wird eine Dentalrestauration, beispielsweise ein Inlay, Onlay, Veneer, eine Krone, Brücke, ein Gerüst oder eine Dentalkeramik, in den präparierten Zahn eingebracht oder darauf aufgebracht. Die anschließende Härtung der Zusammensetzung erfolgt vorzugsweise durch Licht und/oder durch Selbsthärtung. Die Dentalrestauration wird hierbei am Zahn befestigt.

Die erfindungsgemäßen Zusammensetzungen können auch als extraorale Werkstoffe (nicht therapeutisch) eingesetzt werden, beispielsweise bei der Herstellung oder Reparatur von Dentalrestaurationen. Sie eignen sich zudem als Materialien zur Herstellung und Reparatur von Inlays, Onlays, Kronen oder Brücken.

Zur Herstellung von Dentalrestaurationen wie Inlays, Onlays, Kronen oder Brücken, wird mindestens eine erfindungsgemäße Zusammensetzung auf an sich bekannte Weise zu der gewünschten Dentalrestauration geformt und dann gehärtet. Die Härtung kann durch Licht, Selbsthärtung oder vorzugsweise thermisch erfolgen.

Bei der Reparatur von Dentalrestaurationen werden die erfindungsgemäßen Zusammensetzungen auf die zu reparierende Restauration aufgebacht, beispielsweise um Lücken auszubessern oder um Bruchstücke zu verkleben, und anschließend gehärtet.

Die Erfindung wird im Folgenden anhand von Figuren und Beispielen näher erläutert.

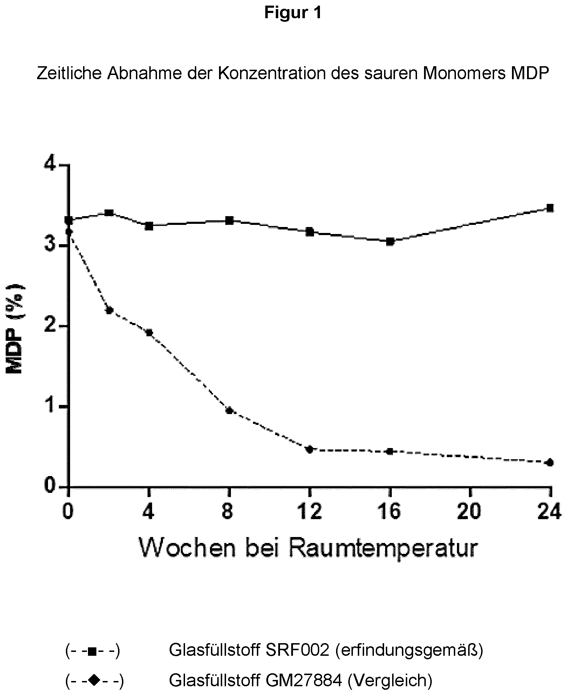

**Figur 1** zeigt die Abnahme der Konzentration des sauren Monomers MDP in Abhängigkeit von der Lagerzeit in Kompositpasten mit einem erfindungsgemäßen säurestabilen röntgenopaken Glasfüllstoff (- -■- -) und einem üblichen röntgenopaken Glasfüllstoff (- -+- -).

### Ausführungsbeispiele

### Beispiel 1

### Herstellung von säurestabilen Füllstoffen

Zur Herstellung der Glasfüllstoffe wurde eine homogene Mischung von ca. 120 g der in Tabelle 1 genannten Oxiden in einem Labormischgerät (SpeedMixer^{®}, Hauschild GmbH & Co. KG, Hamm) hergestellt. Danach wurde die Rohmaterialmischung bei 1650°C in einem Pt/Rh10-Tiegel erschmolzen und 1,5 h homogenisiert. Anschließend wurde die Glasschmelze zur Herstellung einer Glasfritte in Wasser abgeschreckt. Die Zerkleinerung der Glasfritte erfolgte mittels einer Gegenstrahlmühle bis zu einer mittleren Partikelgröße von D₅₀ von ca. 5 µm. Die Zusammensetzung der hergestellten Gläser SFR001 bis SFR005 ist in Tabelle 1 dargestellt.

**Tabelle 1: Zusammensetzung (Gew.-%) der Gläser SRF001-005**

| **Oxid/Glasfüllstoff** | **001** | **002** | **003** | **004** | **005** |
|---|---|---|---|---|---|
| SiO₂ | 58,0 | 58,0 | 58,0 | 58,0 | 58,0 |
| Al₂O₃ | 14,0 | 16,0 | 12,0 | 18,0 | 20,0 |
| B₂O₃ | 6,0 | 4,0 | 8,0 | 2,0 | 0,0 |
| La₂O₃ | 1,6 | 1,6 | 1,6 | 1,6 | 1,6 |
| WO₃ | 10,0 | 10,0 | 10,0 | 10,0 | 10,0 |
| ZrO₂ | 2,0 | 2,0 | 2,0 | 2,0 | 2,0 |
| MgO | 6,4 | 6,4 | 6,4 | 6,4 | 6,4 |
| CaO | 2,0 | 2,0 | 2,0 | 2,0 | 2,0 |
| Σ | 100,0 | 100,0 | 100,0 | 100,0 | 100,0 |

Zur Silanisierung der Glasfüllstoffe wurden die Füllstoffe (90 g) jeweils mit 6 g 3-Methacryloyloxypropyltrimethoxysilan (Silan A-174, Sigma Aldrich) versetzt und anschließend für 15 min gemischt (Turbola-Mischer, Willy A. Bachofen AG). Danach wurden 2,5 g deionisiertes Wasser zugegeben und nochmals für 15 min gemischt. Anschließend wurden die Füllstoffe jeweils durch ein 90-µm-Kunststoffsieb gesiebt, für 24 h ruhen gelassen und dann für 3 Tage im Trockenschrank bei 50°C getrocknet bis kein freies Silan mehr nachweisbar war (Gaschromatographie).

### Beispiel 2

### Untersuchung der Lagerstabilität von Kompositpasten auf der Basis des erfindungsgemäßen Füllstoffs SRF002 (silanisiert) und eines üblichen röntgenopaken Glasfüllstoffs 15 GM 27884

Mit dem erfindungsgemäßen Füllstoff SRF002 (silanisiert) aus Beispiel 1 und einem röntgenopaken, dentalen Glasfüllstoff (GM 27884, Firma Schott, mittlere Partikelgröße 1 µm, spezifische Oberfläche (BET DIN ISO 9277) 3,9 m²/g, Zusammensetzung (Gew.-%): Al₂O₃: 10, B₂O₃: 10, BaO: 25 und SiO₂: 55) wurden Kompositpasten hergestellt. Die Zusammensetzungen (Gew.-%) der Kompositpasten sind in Tabelle 2 angegeben.

**Tabelle 2: Zusammensetzung der Kompositpasten (Gew.-%)**

| **Bestandteil/Komposit** | **EC52-0981** | **EC31-0221*)** |
|---|---|---|
| MDP¹⁾ | 3,50 | 3,29 |
| TEGDMA²⁾ | 11,37 | 18.68 |
| NK-Ester 9G³⁾ | 2,53 | 2,37 |
| V-392⁴⁾ | 15,57 | 14,63 |
| BHT⁵⁾ | 0,03 | 0,03 |
| Glasfüllstoff SRF002 sil. (Bsp. 1) | 65,00 | - |
| Glasfüllstoff GM27884 sil. | - | 65,00 |
| Aerosil 200⁶⁾ | 2,00 | - |
| HDK 2000⁷⁾ | - | 4,00 |

| | | |
|---|---|---|
| *) Vergleichsbeispiel ¹⁾ MDP (10-Methacryloyloxydecyldihydrogenphosphat, Orgentis) ²⁾ TEGDMA (Triethylenglycoldimethacrylat) ³⁾ NK-Ester 9G (Polyethylenglycol-400-dimethacrylat, Kowa Europa GmbH) ⁴⁾ V-392 (N,N'-Diethyl-1,3-bis(acrylamido)propan, Ivoclar Vivadent AG) ⁵⁾ BHT (2,6-Di-tert-butyl-p-kresol) ⁶⁾ Aerosil 200, pyrogenes SiO₂, spez. Oberfläche 200 m²/g (Evonik) ⁷⁾ HDK 2000, pyrogenes SiO₂, spez. Oberfläche 200 m²/g (Wacker) | | |

Die Pasten wurden bei Raumtemperatur gelagert, und über einen Zeitraum von mehreren Wochen wurde die zeitliche Veränderung des MDP-Gehalts mittels ³¹P-NMR-Spektroskopie bestimmt. Für die ³¹P-NMR-Messung wurden jeweils 1,5 g der zu analysierenden Kompositprobe in ein Zentrifugenröhrchen eingewogen und mit 1,5 ml einer Lösung von 0,1 g Triphenylphosphin in 10 ml deuteriertem Aceton (Aceton-d₆) als innerem Standard versetzt. Die resultierende Suspension wurde mithilfe eines Plattformschüttlers (Vibramax, Heidolph Instruments GmbH & Co. KG, Schwabach) mind. 5 min geschüttelt und anschließend bei 3500 U/min für 15 min zentrifugiert. Der Überstand wurde mittels Einweg-Pipette in ein NMR Probenröhrchen transferiert. Die ³¹P-NMR-Messung erfolgte mit einem 400 MHz Kernresonanz-Spektrometer (Avance DPX 400, Bruker Spectrospin). Aus dem Verhältnis der Peakflächen in Bezug auf den inneren Standard wurde die MDP-Konzentration berechnet. Fig. 1 zeigt die zeitliche Änderung des MDP-Gehalts. Die Ergebnisse in Fig. 1 belegen eine deutlich verbesserte Lagerstabilität der Paste auf Basis des erfindungsgemäßen Füllstoffs SRF002 (- -■- -) im Vergleich zu dem dentalen Glasfüllstoff GM 27884 (- -◆- -).

## Patentansprüche

1. Röntgenopaker Glasfüllstoff, **dadurch gekennzeichnet, dass** er die folgende Zusammensetzung aufweist (Gew.-%): SiO₂: 54,0 - 64,0; Al₂O₃: 10,0 - 25,0; B₂O₃: 0,0 - 10,0; La₂O₃: 0,1 - 10,0; WO₃: 5,0 - 14,0; ZrO₂: 0,1 -7,0; MgO: 1,0 - 7,9; CaO: 0,1 - 6,0; ZnO: 0 - 6,0; SrO: 0 - 6,0, wobei sich alle Prozentangaben auf die Gesamtmasse des Glases beziehen und alle Komponenten als Oxide berechnet werden.

2. Glasfüllstoff nach Anspruch 1, der die folgende Zusammensetzung aufweist (Gew.-%): SiO₂: 55,0 - 63,0, bevorzugt 55,0 - 62,0; Al₂O₃: 12,0 - 24,0, bevorzugt 14,0 - 22,0; B₂O₃: 0,1 - 8,0, bevorzugt 0,1 - 5,0; LazOs: 0,1 - 8,0, bevorzugt 0,1 - 6,0; WO₃: 5,0 - 13,0, bevorzugt 5,0 - 12; ZrO₂: 0,1 - 6,0, bevorzugt 0,1 - 5,0; MgO: 2,0 - 7,5, bevorzugt 3,0 - 7,0; CaO: 0,1 - 4,0, bevorzugt 0,1 - 3,0; ZnO: 0-6,0, bevorzugt 0-4,0; SrO: 0-6,0., bevorzugt 0-5,0, wobei sich alle Angaben auf die Gesamtmasse des Glases beziehen und alle Komponenten als Oxide berechnet werden.

3. Glasfüllstoff nach Anspruch 1 oder 2, der keine Alkalimetalloxide und vorzugsweise auch kein BaO enthält.

4. Glasfüllstoff nach einem der Ansprüche 1 bis 3, der nicht oberflächenbehandelt ist.

5. Radikalisch polymerisierbare Zusammensetzung, die mindestens ein radikalisch polymerisierbares Monomer ohne Säuregruppe, mindestens ein säuregruppenhaltiges radikalisch polymerisierbares Monomer, einen Glasfüllstoff und mindestens einen Initiator für die radikalische Polymerisation enthält, **dadurch gekennzeichnet, dass** sie mindestens einen Glasfüllstoff gemäß einem der Ansprüche 1 bis 4 enthält.

6. Zusammensetzung nach Anspruch 5, die
a) 10 bis 80 Gew.-%, bevorzugt 20 bis 75 Gew.-% und besonders bevorzugt 30 bis 70 Gew.-% des röntgenopaken Glasfüllstoffs,
b) ggf. 0,1 bis 25 Gew.-%, bevorzugt 1 bis 20 Gew.-% und besonders bevorzugt 2 bis 15 Gew.-% eines oder mehrerer weiterer Füllstoffe,
c) 1 bis 15 Gew.-%, bevorzugt 2 bis 12 Gew.-% und besonders bevorzugt 3 bis 10 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,1 bis 8 Gew.-%, bevorzugt 0,5 bis 6 Gew.-% und besonders bevorzugt 1 bis 5 Gew.-% eines Initiators für die radikalische Polymerisation,
h) 0 bis 20 Gew.-%, bevorzugt 0,2 bis 10 Gew.-% und besonders bevorzugt 1 bis 7 Gew.-% Wasser und
i) 0,01 bis 5 Gew.-%, bevorzugt 0,1 bis 3 Gew.-% und besonders bevorzugt 0,1 bis 2 Gew.-% eines oder mehrerer Additive enthält, jeweils bezogen auf die Gesamtmasse der Zusammensetzung.

7. Zusammensetzung nach einem der Ansprüche 5 bis 6, die als radikalisch polymerisierbares Monomer (d) mindestens ein polyfunktionelles Monomer enthält, das aus Bisphenol-A-dimethacrylat, Bis-GMA (ein Additionsprodukt aus Methacrylsäure und Bisphenol-A-diglycidylether), ethoxy- oder propoxylierten Bisphenol-A-dimethacrylaten, wie Bisphenol-A-dimethacrylat mit 3 Ethoxygruppen oder 2,2-Bis[4-(2-methacryloyloxypropoxy)phenyl]propan, UDMA (Additionsprodukt aus 2-Hydroxyethylmethacrylat und 2,2,4-Trimethylhexamethylen-1,6-diisocyanat), Tetramethylxylylendiurethanethylenglycoldi(meth)acrylat,. Tetramethylxylylendiurethan-2-methylethylenglykoldiurethandi(meth)acrylat (V-380), Di-, Tri- oder Tetraethylenglycoldimethacrylat, Trimethylolpropantrimethacrylat, Pentaerythrittetramethacrylat, Glycerindi- und Glycerintrimethacrylat, 1,4-Butandioldimethacrylat, 1,10-Decandioldimethacrylat (D₃MA), Bis(methacryloy-Ioxymethyl)tricyclo-[5.2.1.0_{2,6}]decan (DCP), Polyethylenglycol- oder Polypropylenglycoldimethacrylaten, wie Polyethylenglycol-200-dimethacrylat (PEG-200-DMA) oder Polyethylenglycol-400-dimethacrylat (PEG-400-DMA), 1,12-Dodecandioldimethacrylat, Urethanen aus 2-(Hydroxymethyl)acrylsaure und Diisocyanten, wie 2,2,4-Trimethylhexamethylendiisocyanat oder Isophorondiisocyanat, Pyrrolidonen, wie 1,6-Bis(3-vinyl-2-pyrrolidonyl)hexan, Bisacrylamiden, wie Methylen- oder Ethylenbisacrylamid, Bis(meth)acrylamiden, wie N,N'-Diethyl-1,3-bis(acrylamido)-propan, 1,3-Bis(methacrylamido)propan, 1,4-Bis(acrylamido)butan, 1,4-Bis(acryloyl)piperazin und Mischungen davon ausgewählt ist.

8. Zusammensetzung nach einem der Ansprüche 5 bis 7, die als säuregruppenhaltiges Monomer (c) mindestens ein Monomer mit einem pKa-Wert von 0,5 bis 4,0, besonders bevorzugt 1,0 bis 3,5 und ganz besonders bevorzugt 1,5 bis 2,5 bei Raumtemperatur enthält.

9. Zusammensetzung nach einem der Ansprüche 5 bis 8, die als säuregruppenhaltiges Monomer (c) mindestens ein Monomer enthält, das aus Monomeren, die eine Phosphorsäureestergruppe oder Phosphonsäuregruppe enthalten, vorzugsweise 2-Methacryloyloxyethylphenylhydrogenphosphat, 10-Methacryloyloxydecyldihydrogenphosphat (MDP) Glycerindimethacrylatdihydrogenphosphat, Dipentaerythritpentamethacryloyloxyphosphat, 4-Vinylbenzylphosphonsäure, 2-[4-(Dihydroxyphosphoryl)-2-oxa-butyl]acrylsäure, 2-[4-(Dihydroxyphosphoryl)-2-oxa-butyl]-acrylsäure-2,4,6-trimethylphenylester, 4-(Meth)acryloyloxyethyltrimellitsäureanhydrid, 10-Methacryloyloxydecylmalonsäure, N-(2-Hydroxy-3-methacryloyloxypropyl)-N-phenylglycin, 4-Vinylbenzoesäure oder einer Mischung davon ausgewählt ist.

10. Zusammensetzung nach einem der Ansprüche 6 bis 9, die als oligomere Carbonsäure (e) Polyacrylsäure mit einer zahlenmittleren Molmasse kleiner 7200 g/mol, bevorzugt kleiner 7000 g/mol und besonders bevorzugt kleiner 6800 g/mol enthält.

11. Zusammensetzung nach einem der Ansprüche 5 bis 10, die als radikalisch polymerisierbares Monomer (f) mindestens ein monofunktionelles Monomer enthält, das aus Benzyl-, Tetrahydrofurfuryl(meth)acrylat, Isobornyl(meth)acrylat, p-Cumylphenoxyethylenglycolmethacrylat (CMP-1E), 2-([1,1'-Biphenyl]-2-oxy)ethylmethacrylat (MA-836), Tricyclodecanmethyl(meth)acrylat, 2-(2-Biphenyloxy)ethyl(meth)acrylat, 2-Hydroxyethyl(methacrylat), Hydroxyethylpropyl-(methacrylat), 2-Acetoxyethylmethacrylat oder einer Mischung davon ausgewählt ist.

12. Zusammensetzung nach einem der Ansprüche 5 bis 11, die eine Katalysatorpaste und eine Basis-Paste umfasst, wobei die Katalysatorpaste
a) 10 bis 80 Gew.-%, bevorzugt 20 bis 75 Gew.-% und besonders bevorzugt 30 bis 70 Gew.-% des röntgenopaken Glasfüllstoffs,
b) ggf. 0,1 bis 25 Gew.-%, bevorzugt 1 bis 20 Gew.-% und besonders bevorzugt 2 bis 15 Gew.-% eines oder mehrerer weiterer Füllstoffe,
c) 2 bis 30 Gew.-%, bevorzugt 4 bis 24 Gew.-% und besonders bevorzugt 6 bis 20 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 1 bis 20 Gew.-%, bevorzugt 2 bis 15 Gew.-% und besonders bevorzugt 3 bis 10 Gew.-% eines oder mehrerer monofunktioneller Monomere,
g) 0,01 bis 16 Gew.-%, bevorzugt 0,2 bis 12 Gew.-% und besonders bevorzugt 0,5 bis 10 Gew.-% mindestens eines Peroxids und/oder Hydroperoxids ggf. mindestens eines Photoinitiators,
h) 0 bis 20 Gew.-%, bevorzugt 0,2 bis 10 Gew.-% und besonders bevorzugt 1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive enthält, jeweils bezogen auf die Gesamtmasse der Katalysatorpaste,
und wobei die Basis-Paste
a) 10 bis 80 Gew.-%, bevorzugt 20 bis 75 Gew.-% und besonders bevorzugt 30 bis 70 Gew.-% des röntgenopaken Glasfüllstoffs,
b) ggf. 0,1 bis 25 Gew.-%. bevorzugt 1 bis 20 Gew.-% und besonders bevorzugt 2 bis 15 Gew.-% eines oder mehrerer weiterer Füllstoffe,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,01 bis 16 Gew.-%, bevorzugt 0,3 bis 12 Gew.-% und besonders bevorzugt 1 bis 10 Gew.-% mindestens eines geeigneten Reduktionsmittels und ggf. mindestens eines Photoinitiators,
h) 0 bis 20 Gew.-%, bevorzugt 0,2 bis 10 Gew.-% und besonders bevorzugt 1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive enthält, jeweils bezogen auf die Gesamtmasse der Basis-Paste.

13. Zusammensetzung nach einem der Ansprüche 5 bis 12 zur therapeutischen Anwendung als Dentalwerkstoff, vorzugsweise als dentaler Zement, Beschichtungs- oder Verblendmaterial, Füllungskomposit oder Befestigungszement.

14. Nicht-therapeutische Verwendung einer Zusammensetzung gemäß einem der Ansprüche 5 bis 12 zur Herstellung oder Reparatur von Dentalrestaurationen, insbesondere von Inlays, Onlays, Kronen oder Brücken.

15. Verendung eines röntgenopaken Glases gemäß einem der Anspruche 1 bis 4 zur Herstellung eines Dentalwerkstoffs.
